(19) Europäisches Patentamt
European Patent Office
Office européen des brevets

(11) Veröffentlichungsnummer: **0 465 900 B1**

(12) **EUROPÄISCHE PATENTSCHRIFT**

(45) Veröffentlichungstag der Patentschrift: **06.09.95**

(51) Int. Cl.[6]: **C07D 263/04**, C08G 18/38

(21) Anmeldenummer: **91110324.0**

(22) Anmeldetag: **22.06.91**

# (54) Im wesentlichen aus Bisoxazolanen bestehende Oxazolangemische, ein Verfahren zu deren Herstellung und ihre Verwendung als Härter für Isocyanatgruppen aufweisende Kunststoffvorläufer.

(30) Priorität: **07.07.90 DE 4021659**

(43) Veröffentlichungstag der Anmeldung:
**15.01.92 Patentblatt 92/03**

(45) Bekanntmachung des Hinweises auf die Patenterteilung:
**06.09.95 Patentblatt 95/36**

(84) Benannte Vertragsstaaten:
**AT BE CH DE ES FR GB IT LI NL SE**

(56) Entgegenhaltungen:
**EP-A- 0 228 935**
**EP-A- 0 260 339**
**EP-A- 0 329 375**
**DE-A- 2 446 438**

(73) Patentinhaber: **BAYER AG**

**D-51368 Leverkusen (DE)**

(72) Erfinder: **Blum, Harald, Dr.**
**Auf dem Westkamp 1**
**W-4175 Wachtendonk 1 (DE)**
Erfinder: **Pedain, Josef, Dr.**
**Haferkamp 6**
**W-5000 Köln 80 (DE)**
Erfinder: **Hentschel, Karl-Heinz, Dr.**
**Friedrich-Offermann-Strasse 57c**
**W-5060 Bergisch Gladbach 1 (DE)**

EP 0 465 900 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue, im wesentlichen aus Bisoxazolanen bestehende Oxazolangemische, ein Verfahren zu deren Herstellung und ihre Verwendung als Härter für Isocyanatgruppen aufweisende Kunststoffvorläufer.

Verbindungen mit Oxazolanstruktureinheiten I

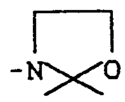

(I),

der Einfachheit halber als Oxazolane bezeichnet, haben die Eigenschaft durch Einwirken von z.B. Luftfeuchtigkeit in die Ausgangskomponenten - Hydroxyamin und Carbonylverbindung - rückzuspalten.

Derartige Verbindungen stellen daher potentielle Reaktionspartner für Isocyanatgruppen aufweisende Kunststoffvorläufer, d.h. insbesondere organische Polyisocyanate oder Isocyanatgruppen aufweisende Prepolymere dar. In der DE-PS 24 46 438 werden Urethangruppen enthaltende Oxazolane beschrieben, die frei von Estergruppen sind und deshalb eine erhöhte Hydrolysestabilität, insbesondere im alkalischen Bereich aufweisen. Diese Oxazolane können z.B. mit Isocyanatgruppen aufweisenden Prepolymeren auf Basis von aliphatischen Diisocyanaten zu Einkomponenten-Formulierungen verarbeitet werden, die gut lagerfähig sind und mit Feuchtigkeit zu vernetzten Beschichtungen aushärten.

Oxazolane auf Basis von Bishydroxyaminen und Aldehyden lassen sich besonders leicht herstellen und zeigen ein ausgewogenes Verhältnis von Reaktivität und Lagerstabilität.

Die in der DE-PS 24 46 438 beschriebenen, insbesondere alle in den Beispielen eingesetzten Oxazolane auf Aldehydbasis sind jedoch noch mit einigen Nachteilen behaftet:

- Die genannten Oxazolane des Standes der Technik weisen eine ausgeprägte Neigung auf, bei Lagerung in einen kristallinen Zustand überzugehen, was naturgemäß erhebliche Probleme bei Lagerung, Transport und Applikation mit sich bringt. Es müssen entweder Lö-sungsmittel zugesetzt werden, was aus ökologischen und ökonomischen Gesichtspunkten unerwünscht ist, oder die Produkte müssen oberhalb Raumtemperatur also in beheizten Räumen gelagert werden, was ebenfalls von erheblichem praktischem Nachteil ist.
- Die genannten Oxazolane des Standes der Technik weisen bei Raumtemperatur Viskositäten von über 15 000 mPa.s auf, so daß bei ihrer Herstellung und Verarbeitung der aus ihnen hergestellten Beschichtungsmittel häufig reaktive Verdünner und/oder flüchtige Lösungsmittel mitverwendet werden müssen.
- Die genannten Oxazolane des Standes der Technik enthalten leicht flüchtige Aldehyde, die bei Applikation von entsprechenden Formulierungen aufgrund ihrer hohen Flüchtigkeit und ihres stechenden Geruchs zu unwillkommenen Belästigungen führen können.

Überraschenderweise wurde jetzt gefunden, daß die erfindungsgemäßen, nachstehend näher beschriebenen Oxazolangemische mit diesen Nachteilen nicht behaftet sind. Durch die Bereitstellung der erfindungsgemäßen Oxazolangemische stehen nunmehr ohne Geruchsbelästigung verarbeitbare, niedrigviskose, lösungsmittelfreie Produkte zur Verfügung die auch nach Lagerung bei niedrigen Temperaturen keine Kristallisationsneigung zeigen, und die ausgezeichnet als Härter für Isocyanatgruppen aufweisende Kunststoffvorläufer geeignet sind.

Gegenstand der Erfindung sind auch als Härter für Isocyanatgruppen aufweisende Kunststoffvorläufer geeignete Oxazolangemische, die im wesentlichen aus

a) aus Bisoxazolanen der soeben genannten Formel (II)

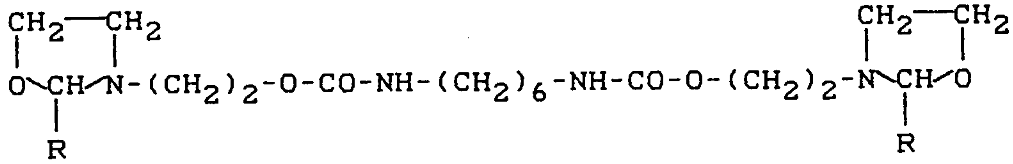

(II)

und

b) Verbindungen der Formel (III)

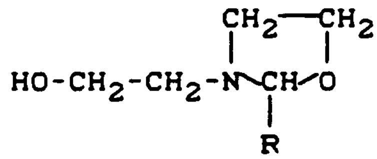

(III)

bestehen, wobei in diesen Formeln R jeweils für einen gesättigten, verzweigten, aliphatischen Kohlenwasserstoffrest mit 5 bis 8 Kohlenstoffatomen steht, und wobei die Menge der Komponente b) 0,03 bis 0,3 Mol pro Mol der Komponente a) beträgt.

Gegenstand der Erfindung ist auch ein Verfahren zur Herstellung dieser Oxazolangemische, welches dadurch gekennzeichnet ist, daß man

a) in einer ersten Reaktionsstufe Diethanolamin mit 1,01 bis 1,5 Mol, pro Mol Diethanolamin, eines Aldehyds der Formel (IV)

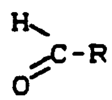

(IV)

gegebenenfalls unter Einsatz eines Schleppmittels bei 60 bis 160 °C zu einem Monohydroxyoxazolan der Formel (III) umsetzt, nach der Umsetzung den überschüssigen Aldehyd und Schleppmittel destillativ abtrennt und

b) in einer zweiten Stufe 1,6-Diisocyanatohexan mit 2,03 bis 2,3 Mol, pro Mol 1,6-Diisocyanatohexan, des gemaß erster Stufe erhaltenen Oxazolans der Formel (III) zur Reaktion bringt, bis im Reaktionsgemisch keine Isocyanatgruppen mehr nachweisbar sind.

Gegenstand der Erfindung ist schließlich auch die Verwendung der genannten Oxazolangemische als Härter für Isocyanatgruppen aufweisende Kunststoffvorläufer.

Die EP-A-0 329 375 beschreibt ein 2-komponentig zu verarbeitendes System, welches 4 Bindemittelkomponenten A) - D) enthält, wobei neben einer vernetzungsfähigen Polysiloxan-Komponente A), einem Härter B) für die Komponente A) auch Polyisocyanate C) und als Härter für die Komponente C) geeignete Bisoxazolidine genannt werden. Bei diesen Bisoxazolidinen D) kann es sich u.a. auch um solche der obengenannten Formel (II) mit R = $-C_4H_9$ handeln. Die Vorveröffentlichung beschreibt jedoch an keiner Stelle, daß Gemische von Bisoxazolidinen der Formel (II) mit der erfindungswesentlichen Bedeutung von R mit Hydroxyethyloxazolidinen der Formel (III) besonders niedrigviskose Härter für Isocyanatgruppen aufweisende Kunststoffvorläufer darstellen, bei denen die Anwesenheit der Monooxazolidine der Formel (III) den Härtungsprozeß nicht stört und insbesondere, daß die erfindungsgemäßen Gemische auch bei winterlichen Temperaturen (3 °C) keinerlei Tendenz zur Kristallisation zeigen.

Ausgangsmaterialien zur Herstellung der Hydroxyethyloxazolane der Formel (III) sind Diethanolamin und Aldehyde der Formel (IV), wobei in dieser Formel R für einen gesättigten, verzweigten, aliphatischen Kohlenwasserstoffrest mit 5 bis 8, vorzugsweise mit 7 Kohlenstoffatomen steht. Geeignete Aldehyde der Formel (IV) sind beispielsweise 2-Ethylhexanal, 2-Methylpentanal, 2,3-Dimethylpentanal, 2-Methylheptanal, 2-Methylhexanal, 2-Ethylpentanal, wobei 2-Ethylhexanal besonders geeignet ist.

Zur Herstellung der Hydroxyethyloxazolane der Formel (III), d.h. bei der Durchführung der ersten Stufe des erfindungsgemäßen Verfahrens werden Aldehyd und Diethanolamin im allgemeinen unter Verwendung eines Aldehyd-Überschusses, d.h. unter Verwendung von Aldehyd und Diethanolamin im Molverhältnis von 1,01:1 bis 1,5:1, vorzugsweise 1,02:1 bis 1,15:1 eingesetzt.

Die bei der Umsetzung ablaufende Wasserabspaltung kann unter Zuhilfenahme von geeigneten Schleppmitteln wie Isooctan, Cyclohexan, Toluol, Xylol oder aliphatische und/oder aromatische Kohlenwasserstoffgemische durchgeführt werden, es kann jedoch auch überschüssiger Aldehyd als Schleppmittel eingesetzt werden.

Die Wasserabspaltung wird üblicherweise bei Temperaturen von 60 bis 160 °C, gegebenenfalls auch unter Anlegen eines leichten Vakuums (beispielsweise 15 bei 200 mbar) solange durchgeführt, bis die theoretische Wassermenge vollständig und nahezu vollständig abgespalten ist.

Schleppmittel und überschüssiges Blockierungsreagenz (Aldehyd) werden dann durch Anlegen eines Vakuums (beispielsweise 10 bis 100 mbar) aus dem Reaktionsgemisch entfernt.

Werden besonders hohe Anforderungen an die Qualität des Produktes bezüglich Farbzahl und Reinheit gelegt, so kann diese Oxazolanvorstufe auch destillativ gereinigt werden. Die Produkte sieden bei einem Druck von 0,5 bis 2,5 mbar bei ca. 90 bis 120 °C.

In der zweiten Reaktionsstufe des erfindungsgemäßen Verfahrens wird die so erhaltene Oxazolanvorstufe (Hydroxyethyloxazolan der Formel (III) mit 1,6-Diisocyanatohexan umgesetzt, wobei im allgemeinen unter Einhaltung eines Molverhältnisses von Hydroxyethyloxazolan zu 1,6-Diisocyanatohexan von 2,03:1 bis 1,3:1, vorzugsweise 2,04:1 bis 2,22:1 gearbeitet wird.

Bei dieser Umsetzung entstehen erfindungsgemäße Oxazolangemische, die im wesentlichen aus erfindungsgemäßen Bisoxazolanen der vorstehend genannten Formel (II) und in untergeordnetem Umfang aus überschüssigem Hydroxyethyloxazolan der Formel (III) bestehen, wobei in den Gemischen pro Mol Bisoxazolan der Formel (II) 0,03 bis 0,3 Mol, vorzugsweise 0,04 bis 0,22 Mol Hydroxyethyloxazolan der Formel (III) vorliegen.

Die Umsetzung in der zweiten Stufe des erfindungsgemäßen Verfahrens wird im allgemeinen innerhalb des Temperaturbereichs von 50 bis 100 °C durchgeführt und kann gegebenenfalls durch Zugabe geeigneter Katalysatoren wie beispielsweise Dibutylzinnoxid oder Dibutylzinndilaurat beschleunigt werden.

Der geringe Überschuß an Hydroxyethyloxazolan der Formel (III) in den erfindungsgemäßen Gemischen stört deren erfindungsgemäße Verwendung nicht, so daß bei der Durchführung des erfindungsgemäßen Verfahrens unter Verwendung des genannten Überschusses an Hydroxyethyloxazolan der Formel (III) gegenüber 1,6-Diisocyanatohexan gearbeitet wird, um sicherzustellen, daß das resultierende Umsetzungsprodukt frei von Isocyanatgruppen ist.

Falls die Umsetzung in der ersten Stufe des erfindungsgemäßen Verfahrens nicht bis zum völligen Verbrauch des eingesetzten Diethanolamins erfolgte, kann das in der zweiten Stufe eingesetzte Hydroxyethyloxazolan der Formel (III) noch geringe Mengen an Diethanolamin enthalten, die jedoch im allgemeinen tolerierbar sind, da sie mit dem in der zweiten Stufe eingesetzten Diisocyanat zu Harnstoffen abreagieren, die wegen ihrer geringen Konzentration die Verwendbarkeit der erfindungsgemäßen Oxazolangemische nicht beeinträchtigen. Da im allgemeinen die erste Stufe des erfindungsgemäßen Verfahrens bis zur Beendigung der Wasserabscheidung durchgeführt wird, sind die erfindungsgemäßen Oxazolangemische im wesentlichen jedoch frei von derartigen Nebenprodukten.

Die erfindungsgemäßen Oxazolangemische stellen lösungsmittelfreie Härter für Isocyanatgruppen aufweisende Kunststoffvorläufer dar, die im allgemeinen bei 23 ° C eine Viskosität von unter 8000, vorzugsweise von unter 6500 mPa.s aufweisen. In Kombination mit Isocyanatgruppen aufweisenden Kunststoffvorläufern eignen sie sich zur Herstellung von lösungsmittelfreien oder -armen Einkomponenten-Systemen, die ihrerseits als Bindemittel für hochwertige Lacke, Beschichtungsmittel oder Dichtmassen geeignet sind. Die Aushärtung dieser Systeme erfolgt im allgemeinen nach ihrer Applikation unter Zutritt von Luftfeuchtigkeit. Zur Herstellung derartiger Formulierungen geeignete, Isocyanatgruppen aufweisende Kunststoffvorläufer sind beispielsweise organische Polyisocyanate oder Isocyanatprepolymere der in DE-PS 24 46 438 beispielhaft genannten Art.

Bei der erfindungsgemäßen Verwendung können die erfindungsgemäßen Bisoxazolane der Formel (II) oder die erfindungsgemäßen Oxazolangemische auch in Abmischung mit analogen Bisoxazolanen zur Anwendung gelangen, die durch analoge Umsetzung von Hydroxyethyloxazolanen der Formel (III) mit anderen Diisocyanaten als 1,6-Diisocyanatohexan hergestellt worden sind. Derartige Gemische werden beispielsweise dann erhalten, wenn analog der Durchführung des erfindungsgemäßen Verfahrens anstelle von 1,6-Diisocyanatohexan Gemische dieses Diisocyanats mit anderen Diisocyanaten zum Einsatz gelangen. Als andere, hier in Betracht zu ziehende Diisocyanate kommen insbesondere solche mit aliphatisch oder cycloaliphatisch gebundenen Isocyanatgruppen wie beispielsweise Isophorondiisocyanat, Dodecamethylendiisocyanat, Tetramethylendiisocyanat, 1,4-Diisocyanatocyclohexan, 2,4'- und/oder 4,4'-Diisocyanatodicyclohexylmethan oder auch aromatische Diisocyanate wie 2,4- und/oder 2,6-Diisocyanatotoluol in Betracht. Der Anteil dieser anderen Diisocyanate kann bis zu 50 Mol-%, bezogen auf die Gesamtmenge der eingesetzten Diisocyanate betragen. Dementsprechend hoch kann auch der Anteil an analogen Bisoxazolanen auf Basis der zuletztgenannten Diisocyanate liegen. Eine derartige Arbeitsweise bzw. die Verwendung von derartigen Gemischen anstelle der erfindungsgemäßen Bisoxazolane ist jedoch keineswegs bevorzugt.

Beispiele

Alle Reaktionen werden unter $N_2$-Inertgasatmosphäre durchgeführt.

1a) In einem 4-1-Reaktionsgefäß mit Rühr-, Kühl- und Heizvorrichtung und Wasserabscheider werden 1260 g (12 Mol) Diethanolamin und 738 g Isooctan vorgelegt und in 1 Stunde 1690 g (13,2 Mol) 2-Ethylhexanal zudosiert. Die Reaktionsmischung wird am Wasserabscheider solange erhitzt, bis 212 g Wasser (Theorie: 216 g) abgeschieden sind. Anschließend wird bei 70 °C und ca. 10 Torr überschüssiges 2-Ethylhexanal und Isooctan abdestilliert.

Man erhält das entsprechende Hydroxyethyloxazolan 1a) der Formel (III) (R = 1-Ethylpentyl).

1b) In einem Reaktionsgefäß werden 678,9 g (3,15 Mol) des Hydroxyethyloxazolans 1a) vorgelegt, auf 90 °C erwärmt und dann 247 g (1,47 Mol) 1,6-Diisocyanatohexan zudosiert. Die Reaktionsmischung wird solange gerührt, bis im IR-Spektrum keine NCO-Bande mehr erkennbar ist.

Man erhält ein erfindungsgemäßes Oxazolangemisch in Form einer hellgelben Flüssigkeit mit einer Viskosität bei 23 °C von 4300 mPa.s. Die Hauptkomponente besteht aus einem erfindungsgemäßen Bisoxazolan der Formel (II) (R = 1-Ethylpentyl).

2b) 716,7 g (3,33 Mol) der in 1a) erhaltenen Vorstufe werden wie unter 1b) beschrieben mit 252 g (1,5 Mol) 1,6-Diisocyanatohexan umgesetzt. Man erhält so ein erfindungsgemäßes Oxazolangemisch 2b) als hellgelbe Flüssigkeit mit einer Viskosität von 3500 mPa.s bei 23 ° C.

3b) 657,9 g (3,06 Mol) der in 1a) erhaltenen Vorstufe werden wie unter 1b) beschrieben mit 252 g (1,5 Mol) 1,6-Diisocyanatohexan umgesetzt.

Man erhält ein erfindungsgemäßes Oxazolangemisch 3b) als hellgelbe Flüssigkeit mit einer Viskosität von 5200 mPa.s bei 23 ° C.

4b) 502 g (2,33 Mol) der in 1a) erhaltenen Vorstufe werden wie unter 1b) beschrieben mit 141,6 g (0,84 Mol) 1,6-Diisocyanatohexan und 46,6 g (0,21 Mol) Isophorondiisocyanat umgesetzt.

Man erhält auf diese Weise ein Oxazolangemisch welches neben einem erfindungsgemäßen Bisoxazolan der Formel (II) noch eine, der Isophorondiisocyanat-Menge entsprechende Menge eines analogen Bisoxazolans des Isophorondiisocyanats enthält. Das Produkt ist eine hellgelbe Flüssigkeit mit einer Viskosität von 7800 mPa.s bei 23 ° C.

Vergleichsbeispiele

5) (Entsprechend Beispiel 1 der DE-PS 24 46 438). 159 g (1,0 Mol) des aus Diethanolamin und Isobutyraldehyd erhaltenen Hydroxyethyloxazolans der Formel (III) (R = Isopropyl) werden nach Zusatz von 0,1 g Zinn-II-octoat mit 84 g (0,5 Mol) 1,6-Diisocyanatohexan umgesetzt.

Man erhält ein nicht erfindungsgemäßes Bisoxazolan der Formel (II) (R= Isopropyl) als hellgelbe Flüssigkeit mit einer Viskosität von 14 500 mPa.s bei 23 ° C.

6) 324,4 g (2,04 Mol) des in Vergleichsbeispiel 5) beschriebenen Hydroxyethyloxazolans werden mit 168 g (1,0 Mol) 1,6-Diisocyanatohexan zu einer hellgelben Flüssigkeit 6) umgesetzt, die bei 23 ° C eine Viskosität von 11 300 mPa.s aufweist.

7a) Wie in Beispiel 1a) beschrieben werden 174 g (1,66 Mol) Diethanolamin, 94 g Isooctan und 200 g (1,82 Mol) Tetrahydrobenzaldehyd zu einem Hydroxyethyloxazolan 7a) umgesetzt.

7b) 201 g (1,02 Mol) der Vorstufe 7a) werden mit 84 g (0,5 Mol) 1,6-Diisocyanatohexan zu einem nicht erfindungsgemäßen Bisoxazolan der Formel (II)(R = Cyclohexenyl) umgesetzt. Es handelt sich um eine hellbraune Flüssigkeit, die bei 23 ° C eine über 100 000 mPa.s liegende Viskosität aufweist.

8a) Wie in Beispiel 1a) beschrieben werden 420 g (4 Mol) Diethanolamin, 170 g Isooctan und 431 g (4,4 Mol) Cyclohexanon zu einem Hydroxyethyloxazolan 8a) umgesetzt.

8b) 377,5 g (2,04 Mol) der Vorstufe 8a) werden wie in Beispiel 1b) beschrieben mit 168 g (1,0 Mol) 1,6-Diisocyanatohexan zu einem nicht erfindungsgemäßen Bisoxazolan 8b) umgesetzt. Es handelt sich um eine hellgelbe Flüssigkeit, die bei 23 ° C eine Viskosität von 90 000 mPa.s aufweist.

Während die erfindungsgemäßen Oxazolangemische Viskositäten von 3500 bis 7800 mPa.s bei 23 °C aufweisen und damit gut für die Herstellung lösungsmittelfreier Beschichtungssysteme geeignet sind, sind die nach den Vergleichsbeispielen 5) und 6) hergestellten Produkte mit der Viskosität zwischen 11 300 und 14 500 mPa.s nur mit Einschränkung in der Verarbeitbarkeit einsetzbar.

Die nach den Vergleichsbeispielen 7) und 8) hergestellten Produkte sind aufgrund ihrer hohen Viskositäten von ≧90 000 mPa.s nicht zur Herstellung lösemittelfreier Beschichtungen geeignet.

Lagerstabilitätsprüfungen:

Die gemäß den Beispielen 1) bis 4) und den Vergleichsbeispielen 5) bzw. 6) hergestellten Oxazolangemische werden einer Lagerung in geschlossenen Gefäßen bei Raumtemperatur (20-25° C) und bei 3° C unterzogen, dabei wurden folgende Ergebnisse erhalten:

| | Lagerung bei: | |
|---|---|---|
| | Raumtemperatur | 3° C |
| Bsp. 1 | nach 12 Monaten flüssig | nach 12 Monaten flüssig |
| Bsp. 2 | nach 12 Monaten flüssig | nach 12 Monaten flüssig |
| Bsp. 3 | nach 12 Monaten flüssig | nach 12 Monaten flüssig |
| Bsp. 4 | nach 12 Monaten flüssig | nach 12 Monaten flüssig |
| Bsp. 5 | nach 2-3 Monaten durchkristallisiert | nach 1 Woche durchkristallisiert |
| Bsp. 6 | nach 2-3 Monaten durchkristallisiert | nach 1 Woche durchkristallisiert |

Die erfindungsgemäßen Oxazolangemische sind im Gegensatz zu den Vergleichsprodukten lagerstabil und können auch nach längerer Lagerung bei niedrigen Temperaturen sofort verarbeitet werden.

**Patentansprüche**

**1.** Als Härter für Isocyanatgruppen aufweisende Kunststoffvorläufer geeignete Oxazolangemische, dadurch gekennzeichnet, daß sie im wesentlichen aus

a) Bisoxazolanen der in Anspruch 1 genannten Formel (II)

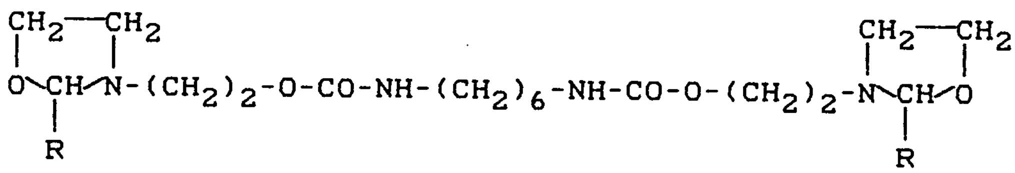

(II)

und

b) Verbindungen der Formel (III)

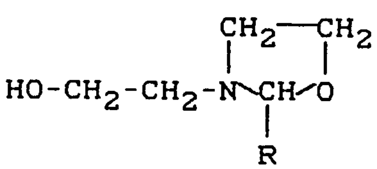

(III)

bestehen, wobei in diesen Formeln R jeweils für einen gesättigten, verzweigten, aliphatischen Kohlenwasserstoffrest mit 5 bis 8 Kohlenstoffatomen steht, und wobei die Menge der Komponente b) 0,03 bis 0,3 Mol pro Mol der Komponente a) beträgt.

**2.** Oxazolangemische gemaß Anspruch 1, dadurch gekennzeichnet, daß der Rest R in den Formeln (II) und (III) jeweils für einen gesättigten, verzweigten, aliphatischen Kohlenwasserstoffrest mit 7 Kohlenstoffatomen steht.

3. Verfahren zur Herstellung von Oxazolangemischen gemaß Anspruch 1, dadurch gekennzeichnet, daß man
   a) in einer ersten Reaktionsstufe Diethanolamin mit 1,01 bis 1,5 Mol, pro Mol Diethanolamin, eines Aldehyds der Formel (IV)

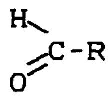

(IV)

   gegebenenfalls unter Einsatz eines Schleppmittels bei 60 bis 160 ° C zu einem Monohydroxyoxazolan der Formel (III) umsetzt, nach der Umsetzung den überschüssigen Aldehyd und Schleppmittel destillativ abtrennt und
   b) in einer zweiten Stufe 1,6-Diisocyanatohexan mit 2,03 bis 2,3 Mol, pro Mol 1,6-Diisocyanatohexan, des gemäß erster Stufe erhaltenen Oxazolans der Formel (III) zur Reaktion bringt, bis im Reaktionsgemisch keine Isocyanatgruppen mehr nachweisbar sind.

4. Verwendung der Oxazolangemische gemaß Anspruch 1 und 2 als Härter für Isocyanatgruppen aufweisende Kunststoffvorläufer.

5. Verwendung gemaß Anspruch 4 in lösungsmittelfreien oder -armen Lacken, Beschichtungs- und Dichtmassen.

**Claims**

1. Oxazolane mixtures suitable as hardeners for plastics precursors containing isocyanate groups, characterized in that they essentially consist of
   a) bis-oxazolanes corresponding to formula (II):

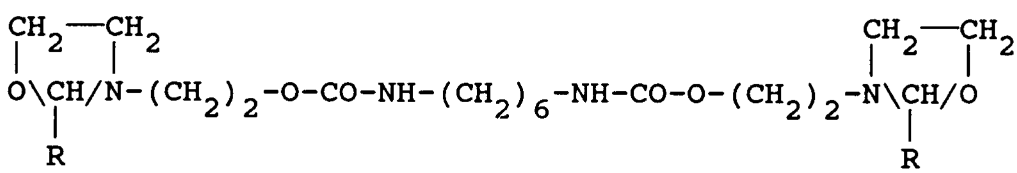

(II)

   and
   b) compounds corresponding to formula (III):

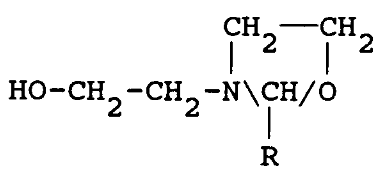

(III)

   in which formulae R is a saturated, branched, aliphatic hydrocarbon radical containing 5 to 8 carbon atoms,
   the quantity of component b) being 0.03 to 0.3 mol per mol component a).

2. Oxazolane mixtures as claimed in claim 1, characterized in that R in formulae (II) and (III) is a saturated, branched, aliphatic hydrocarbon radical containing 7 carbon atoms.

**3.** A process for the production of the oxazolane mixtures claimed in claim 1, characterized in that

a) in a first reaction step, diethanolamine is reacted with 1.01 to 1.5 mol - per mol of diethanolamine - of an aldehyde corresponding to formula (IV):

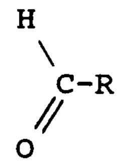

(IV)

at 60 to 160 ° C, optionally in the presence of an entraining agent, to form a monohydroxyoxazolane corresponding to formula (III), the excess aldehyde and entraining agent being removed by distillation after the reaction, and

b) in a second step, 1,6-diisocyanatohexane is reacted with 2.03 to 2.3 mol - per mol of 1,6-diisocyanatohexane - of the oxazolane corresponding to formula (III) obtained in the first step until no more isocyanate groups can be detected in the reaction mixture.

**4.** The use of the oxazolane mixtures claimed in claims 1 and 2 as hardeners for plastics precursors containing isocyanate groups.

**5.** The use claimed in claim 4 in solventless or low-solvent paints, coating compositions and sealing compounds.

## Revendications

**1.** Mélanges d'oxazolannes appropriés comme durcisseurs pour des précurseurs de matières synthétiques présentant des groupes isocyanate, caractérisés en ce qu'ils sont constitués essentiellement par

a) des bisoxazolannes répondant à la formule (II) mentionnée à la revendication 1

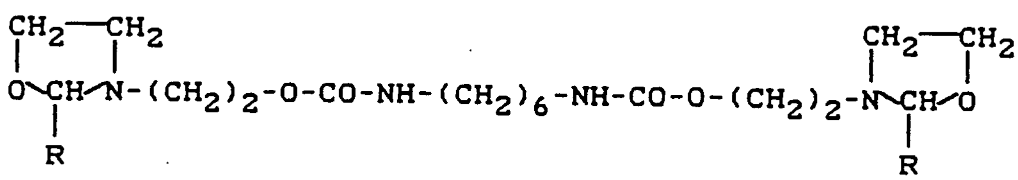

(II)

et

b) des composés de formule (III)

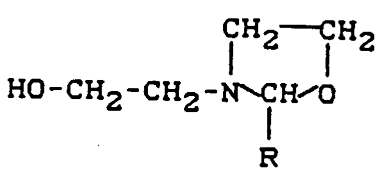

(III)

dans lesquels, dans ces formules, R représente respectivement un radical d'hydrocarbure aliphatique, ramifié, saturé, contenant de 5 à 8 atomes de carbone et dans lesquels la quantité du composant b) s'élève de 0,03 à 0,3 mole par mole du composant a).

**2.** Mélanges d'oxazolannes selon la revendication 1, caractérisés en ce que le radical R dans les formules (II) et (III) représente respectivement un radical d'hydrocarbure aliphatique, ramifié, saturé, contenant 7 atomes de carbone.

**3.** Procédé pour la préparation de mélanges d'oxazolannes selon la revendication 1, caractérisé en ce que

a) dans une première étape réactionnelle, on fait réagir de la diéthanolamine avec de 1,01 à 1,5 mole, par mole de diéthanolamine, d'un aldéhyde de formule (IV)

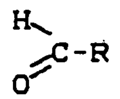

(IV)

éventuellement en mettant en oeuvre un agent d'entraînement à une température de 60 à 160 °C pour obtenir un oxazolanne monohydroxylé répondant à la formule (III); après la mise en réaction, on sépare par distillation l'aldéhyde et l'agent d'entraînement en excès; et

b) dans une seconde étape, on amène à réagir du 1,6-diisocyanatohexane avec de 2,03 à 2,3 moles, par mole du 1,6-diisocyanatohexane, de l'oxazolanne de formule (III) obtenu conformément à la première étape, jusqu'à ce qu'on ne puisse plus détecter de groupes isocyanate dans le mélange réactionnel.

**4.** Utilisation des mélanges d'oxazolannes selon les revendications 1 et 2, comme durcisseurs pour des précurseurs de matières synthétiques présentant des groupes isocyanate.

**5.** Utilisation selon la revendication 4, dans des laques, des vernis ou des peintures et dans des matières d'enduction et d'étanchéification exemptes de ou pauvres en solvants.